Europäisches Patentamt
European Patent Office
Office européen des brevets

(19)

(11) Publication number: **0 324 211 A1**

(12) # EUROPEAN PATENT APPLICATION

(21) Application number: 88300208.1

(22) Date of filing: 12.01.88

(51) Int. Cl.4: A61L 9/01 , //A01N47/44, C11D3/48

(43) Date of publication of application: 19.07.89 Bulletin 89/29

(84) Designated Contracting States: AT BE CH DE ES FR GB GR IT LI LU NL SE

(71) Applicant: S F H AG.
Baldeggstrasse 18
CH-6280 Hochdorf(CH)

(72) Inventor: Gibbs, Anthony
Hurricane Way Fiffers Lane
Norwich NR6 6EW Norfolk(GB)

(74) Representative: Evans, David Charles et al
F.J. CLEVELAND & COMPANY 40-43,
Chancery Lane
London, WC2A 1JQ(GB)

(54) **Improvements in and relating to deodorising compositions.**

(57) The invention provides an odour removal composition characterised by:-

a) a complexing agent selected from a biguanide polymer of the formula (1)

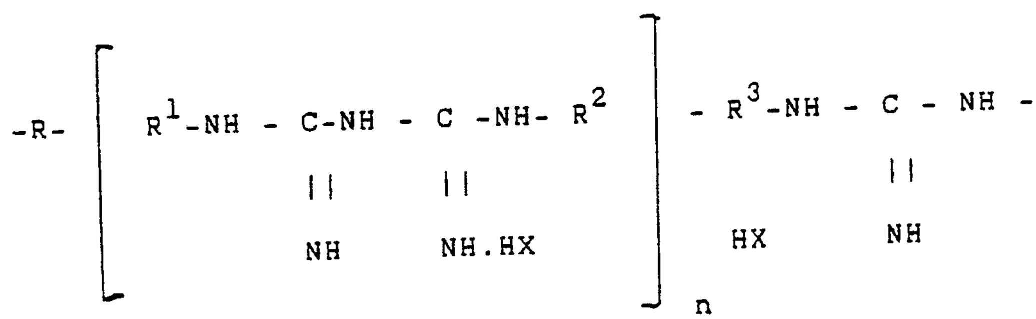

in which R, $R^1$ , $R^2$ and $R^3$ are each a substituted or unsubstituted alkylene group having up to 12 carbon atoms in the main chain, HX is an acid; and n has a value of 2 to 20;

b) a carrier capable of assisting wetting of odour forming compositions; and

c) a cationic moiety; said moiety being part of a chemically independent compound, or chemically associated with a complexing agent or the carrier.

EP 0 324 211 A1

## DEODORISING COMPOSITIONS

This invention relates to deodourising compositions Present technology in connection with odour control has generally been in the field of masking odours rather than actually removing them.

The present invention seeks in a first aspect to provide a broad spectrum deodourising composition.

According to a first aspect therefore of the present invention there is provided an odour removing composition characterised by:-

a) a complexing agent selected from a Biguanide polymer of the formula (1):

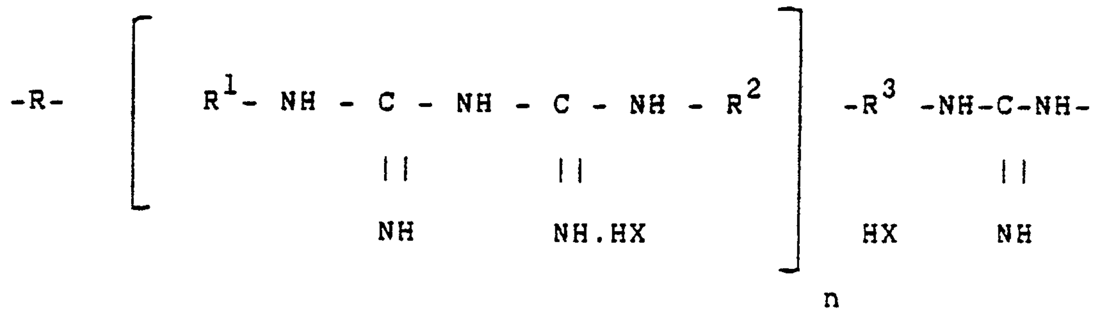

in which R, $R^1$ $R^2$ and $R^3$ are each a substituted or unsubstituted alkylene group having up to 12 carbon atoms in the main chain, HX is an acid; and n has a value of 2 to 20;

b) a carrier capable of assisting wetting of odour forming compositions; and

c) a cationic moiety; said moiety being part of a chemically independent compound, or chemically associated with the complexing agent or the carrier.

Although most compositions can be deodourized using the invention the compositions are less effective on substances such as kerosene for example. The invention is most effective on organic compounds containing hetero atoms, particularly organically bound N and S.

The acid HX is preferably an inorganic acid, most preferably sulphuric or hydrochloric acid. The composition may additionally comprise an auxiliary complexing agent Suitable complexing agents for use in the inventive compositions as hereinbefore set forth may be salts of transition metals, for example the products of a reaction of copper with an organic acid such as lactic, citric, or ascorbic acids.

The deodourising compositions of the invention may thus comprise a complexing agent selected from one or more salts of an organic acid and a transition metal ion capable of oxidation; a carrier capable of assisting wetting of odour forming compositions, and a cationic moiety; said moiety being part of a chemically independent compound, or chemically associated with the carrier or complexing agent.

The compositions of the present invention include an effective amount of a wetting agent as a carrier, which may be cationic and serve as the cationic agent as well. Alternatively, the wetting agent may be a non-ionic or amphoteric detergent carrier, to assist in establishing contact between the active ingredients of the composition of the invention and the article or surface to be deodourised. The compositions of the present invention may in certain circumstances be incorporated within an article such as for example, a foam or sponge plastic of a textile material or the like to provide a more permanent deodourising action. sx

In order to maintain in the formulation pH values providing adequate shelf life and a high level of activity and also to prevent excessive changes of the pH when the formulation comes into contact with acidic or alkaline media, a buffering agent may be added to adjust to and maintain a suitable pH range.

The pH of the concentrated formulation should be between 3 and 12, preferably between 5.5 and 10.

Further, betain compounds may be added to the composition of the present invention; these provide synergistic odour removal compositions. It is a feature of the invention that at least one of the active components may be present as an acid or salt of low solubility in an aqueous media to give slow release properties to the composition.

The precise mechanism whereby the composition in accordance with the present invention operates is not presently understood, but it is thought that the composition seeks to decompose or "attack" the odour forming constituent rather than simply mask the odour. It may do this by forming a complex with the odour forming compositions which reduces volatility, or odour forming abilities, or by chemically interacting with, and thus removing odour forming capability of the odour emitting components.

In a further embodiment of the invention the composition may include an effective amount of selected enzymes capable of degrading odour-emitting compounds.

The composition in accordance with the present invention may be provided in a liquid gel or powder form. When provided in a powder form the constituents of the composition are preferably adsorbed in a carrier powder. A typical carrier powder in accordance with the present invention comprising sodium carbonate and sodium triphosphate; a cross-linked polyacrylamide, pumice dust, or kiselguhr. For many applications the incorporation of the composition into a slow release system has been found a very convenient method for achieving longer term odour elimination. Such slow release systems may comprise a matrix material (which may be porous to increase surface area) into which the composition is incorporated, a receptacle with at least one wall through which the composition will diffuse, or addicts or salts of at least one component of the composition which have reduced solubility so that the rate of release is to the desired level.

In a typical embodiment of the first aspect of the present invention, the composition comprises 20% to 80% by volume of the quaternary ammonium compound and 5% to 40% by volume of the polymer of formula (1) and up to 75% by volume of wetting agent. In the second aspect of the present invention the composition generally comprises up to 10 parts by volume of the quaternary ammonium salt, although higher proportions of up to 50% are possible. The carrier may comprise up to 40 parts by volume while the complexing agent which is present at 100 parts by volume. The compositions may be diluted by up to 500% volume of water. This produces a concentrate which maybe subsequently further diluted prior to use. The extent of dilution will depend on the circumstances and particular application of the composition in accordance with the present invention.

The quaternary ammonium compound is preferably a substituted or unsubstituted aromatic quaternary ammonium salt and is typically a substituted benzyl quaternary ammonium salt. In a specific embodiment of the invention the quaternary ammonium salt is dimethyl benzyl ammonium chloride.

The polymer is preferably a polymeric biguanide having the general formula:

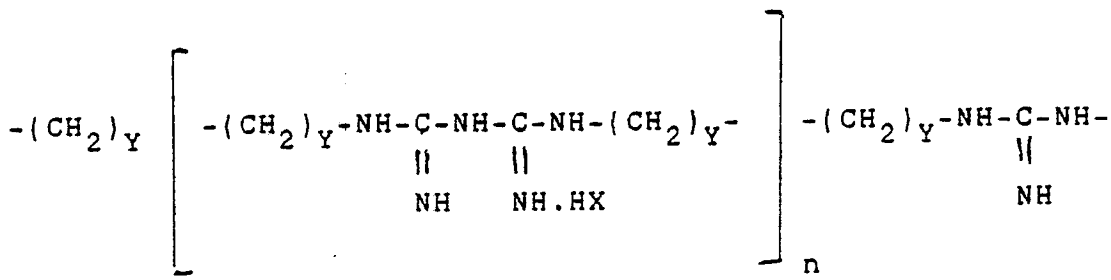

wherein Y is 3 and n is 5 to 15, and HX is an inorganic acid.

The wetting agent may be a cationic surfactant or a nonionic surfactant. Where the wetting agent is a nonionic surfactant it preferably has a Hydrophobic-Lipophilic balance (H.L.B) within the range of 12 to 16 and preferably within the range of 13 to 15. A typical nonionic surfactant for use in accordance with the present invention is nonyl phenol ethylene oxide although it will be appreciated by the man skilled in the art that other surfactants of similar properties may be employed.

The compositions in accordance with the present invention have also been found to have disinfecting properties, especially when used in addition to other known disinfecting agents. The compositions also have fungistatic and even fungicidal properties and are also thought to be effective against, for example, athlete's foot infections around swimming pools, changing rooms and sports areas.

In a powder of spray form they can keep odour under control i sports footwear, while at the same time will reducing the possibility of infection from microorganisms which may develop in such footwear.

The compositions are effective in clinics, hospitals, homes for the aged and other areas where odour control is required, for example, in the case of incontinency problems.

The compositions may be sprayed onto furniture, upholstery fabrics, slippers, clothing or carpets all to prevent odour. In the latter application to carpets, control has been particular effective where pets or babies are known to cause odour problems.

Furthermore, the compositions may be employed in restaurants, hospitals or schools; for wiping tables, furniture rails etc., to remove odour and to inhibit the growth of microorganisms.

Where a cationic surfactant is employed, the compositions have antistatic properties as well as odour control effects which makes them useful for inhibiting the build up of static electricity. The compositions can be introduced into foams and sponges. They are also used for the control of fish smells, toilet odours and

control of damp and mould and bilge odours in boats.

Compositions formulated in accordance with the present invention have a long shelf life and are stable at ambient temperatures. The compositions tend to form water insoluble precipitates with anionic surfactants, soaps strong alkalis and complex phosphates.

The formulation in accordance with the present invention is readily soluble in hot, cold, fresh or salt water and a high degree of hardness has little, if any, depressant effect on the odour control ability.

Following is a description by way of example only of methods of carrying the invention into effect.

## EXAMPLE 1

A liquid formulation in accordance with the present invention was prepared as follows:-

Dimethyl Benzyl Ammonium Chloride or 60% concentrates - 35 litres

Polymeric Biguanide having the general chemical structure:

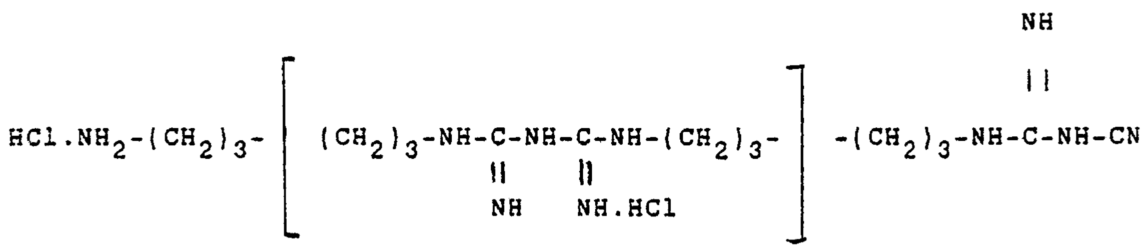

15 litres

Nonyl Phenol Ethylene Oxide - 62.5 litres

Water - 137.5 litres

The required volume of the quaternary ammonium compound, biguanide and surfactant are added to the tank and the required quantity of any marking dye is also added. The total volume is then made up to a total of 250 litres with filtered mains water. The liquid thus formed is stirred until uniform and the resultant liquid composition represents a concentration of approximately 10 times normal use strength.

The composition is then diluted to normal strength and then packed with instruction for use.

## EXAMPLE 2

A composition was prepared as follows:-

| | |
|---|---|
| Dimethyl Benzyl Ammonium Chloride - | 17.5 litres |
| Polymeric Biguanide having the general formula as set forth in Example 1 - | 7.5 litres |
| Nonyl Phenol Ethylene Oxide - | 31.25 litres |

The constituents were introduced into a tank of capacity of 250 litres, a small quantity of dye was

added and water (193.75 litres) was then added to bring the volume up to 250 litres.

20 litres of this composition were added to 40 Kg of sodium carbonate and 20 Kg of sodium tripolyphosphate.

The solid ingredients were introduced into a mixer and the liquid composition was slowly added while mixing. On completion of the introduction of the liquid composition the mixing was carried out for a further 60 minutes and then allowed to cool before passing through a vibrating sieve.

The composition has to be kept under dry conditions.

It has been found that the liquid composition of Example 1 and the powder composition of Example 2 are extremely effective in eliminating odours. The powder composition produced in Example 2 is readily soluble in water, either hot or cold for direct application to on area in which an odour is to be eliminated.

EXAMPLE 3

Various odour-neutralising formulations are shown in Table A, hereinafter set forth. These formulations include a first component formed as follows:-

Polymeric biguanide
Quarternery Ammonium Compound (Dimethyl Benzyl Ammonium Chloride)
Non-ionic surfactant
Water

Depending upon the final product requirement the ratio of Polymer to quaternary compound to surfactant can range, typically, as follows:-

| Basic Formulation | | Polymer | Quaternary | Surfactant |
|---|---|---|---|---|
| (A) | Low detergency/low foam | 5 | 20 | 0 |
| (B) | Medium detergency/low foam | 5 | 40 | 2 |
| (C) | High detergency/medium foam | 5 | 40 | 40 |
| All formulations are by volume. | | | | |

Various formulations are shown in Table 1 which use the above formulations plus other chemicals as noted.

## TABLE 1

### 1. LOW FOAM GLASS WASH

| | |
|---|---|
| Basic formulation (A) | 20% |
| Sodium Hydroxide (40%) | 2% |
| Non-ionic Surfactant | 2% |
| Water to 100% | |

### 2. DEODORIZING WAX POLISH

| | |
|---|---|
| Basic formulation (A) | 10% |
| Fatty Carnauba Wax | 60% |
| Non-ionic Surfactant | 10% |
| Water to 100% | |

### 3. TOILET FLUID

| | |
|---|---|
| Basic formulation (A) | 10% |
| Formaldehyde | 10% |
| Non-ionic Surfactant | 5% |
| Water to 100% | |

4. TOILET FLUID (PERFUMED)

| | |
|---|---|
| Basic formulation (A) | 40% |
| Iso Propyl alcohol | 10% |
| Perfume | 2% |
| Water to 100% | |

5. HIGH TEMPERATURE HIGH PRESSURE SURGICAL INSTRUMENT WASH

| | |
|---|---|
| Basic formulation (A) | 20% |
| Non-ionic Surfactant | 20% |
| Sodium Hydroxide | 2% |
| Anti-foam | As required |
| Water to 100% | |

6. ULTRASONIC CLEANING FLUID

| | |
|---|---|
| Basic formulation (A) | 10% |
| Non-ionic Surfactant | 20% |
| Sodium Hydroxide | 2% |
| Water to 100% | |

7. HIGH PRESSURE WASH FOR SHIPPING CONTAINER

| | |
|---|---|
| Basic formulation (A) | 40% |
| Formaldehyde | 2% |
| Non-ionic Surfactant | 10% |
| Water to 100% | |

8. TOILET BOWL STAIN REMOVER

| | |
|---|---|
| Basic formulation (B) | 10% |
| Hydrochloric Acid | 9% |
| Non-ionic Surfactant | 5% |
| Water to 100% | |

9. BEER LINE AND PUMP CLEANER

| | |
|---|---|
| Basic formulation (B) | 10% |
| Sodium Hydroxide | 12% |
| Non-ionic Surfactant | 10% |
| Water to 100% | |

10. GLASS LIQUOR DISPENSER CLEANER

| | |
|---|---|
| Basic formulation (B) | 5% |
| Non-ionic Surfactant | 10% |

| | |
|---|---|
| Sodium Hydroxide | 5% |
| Water to 100% | |

11. HARD_SURFACE_CLEANER

| | |
|---|---|
| Basic formulation (C) | 60% |
| Sodium Hydroxide | 2% |
| Water to 100% | |

12. HAND_GLASS_WASH_FLUID

| | |
|---|---|
| Basic formulation (C) | 20% |
| Non-ionic Surfactant | 20% |
| Water to 100% | |

13. JANITORIAL_DETERGENT

| | |
|---|---|
| Basic formulation (C) | 40% |
| Non-ionic Surfactant | 20% |
| Water to 100% | |

14. PRE-SOAK_CLEANING_FLUID

| | |
|---|---|
| Basic formulation (C) | 20% |
| Sodium Hydroxide | 5% |
| Non-ionic Surfactant | 10% |
| Water to 100% | |

==========================================================

| BASIC FORMULATION (D) / EXAMPLE 4 | | | |
|---|---|---|---|
| Components | Polymeric Biguanide (20% conc) | - | 5% |
| | Quarternery Ammonium Compound (60% conc) | - | 20% |
| | Non-ionic surfactant (80% conc) | - | 20% |
| | Citric acid (1 mole solution | - | 1% |
| | Ferrous Sulphate (1 mole solution) | - | 10% |
| | Water to | - | 100% |
| All % are by volume. | | | |

## TABLE_2

### 1. GLASS_WASH

| | |
|---|---|
| Basic formulation (D) | 10% |
| Non-ionic Surfactant | 3% |
| Water to 100% | |

2. UPHOLSTERY CLEANER

| | |
|---|---|
| Basic formulation (D) | 20% |
| Non-ionic Surfactant | 20% |
| Isopropyl Alcohol | 5% |
| Water to 100% | |

3. CARPET STAIN REMOVER

| | |
|---|---|
| Basic formulation (D) | 50% |
| Citirc acid (10 mole) | 15% |
| Water to 100% | |

4. PLASTIC CLEANER

| | |
|---|---|
| Basic formulation (D) | 20% |
| Non-ionic Surfactant | 10% |
| Citric acid (10 mole) | 10% |
| Anti-foam | As required |
| Water to 100% | |

EXAMPLE 5

An odour elimination concentrate was prepared as follows:-

| | |
|---|---|
| - Polymeric biguanide | 2% |
| - Quaternary ammonium compound (dimethyl benzylammoniumchloride | 2% |
| - Ascorbic acid | 5% |
| - Ferrous sulphate | 0.1 mole on molar concentration of ascorbic acid |
| - Water to (pH adjusted to 6.5) | 100% |

A diluted solution of this formulation (1 part of solution in 25 parts of water) was used for removing odour from ice boxes, the solution being applied to a sponge, which was used to wipe the interior of the ice boxes.

## Claims

1. An odour removal composition comprising
a) a complexing agent selected from a biguanide polymer of the formula (1)

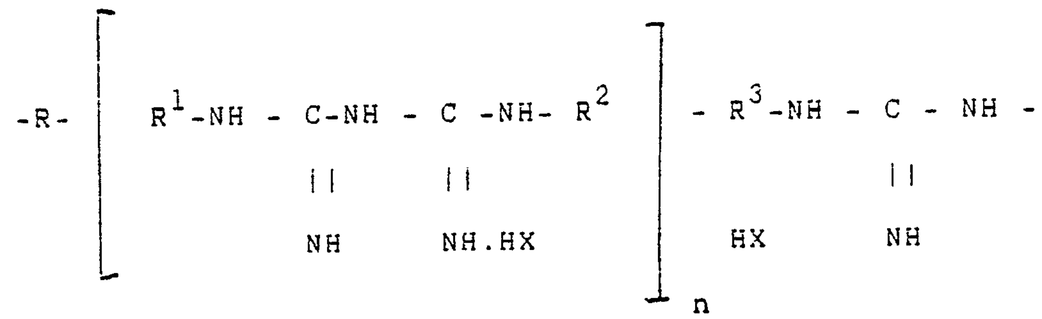

in which R, $R^1$, $R^2$ and $R^3$ are each a substituted or unsubstituted alkylene group having up to 12 carbon atoms in the main chain, HX is an acid; and n has a value of 2 to 20;
b) a carrier capable of assisting wetting of odour forming compositions; and
c) a cationic moiety; said moiety being part of a chemically independent compound, or chemically associated with a complexing agent or the carrier.

2. A composition according to claim 1 characterised in that R to $R^3$ are substituted or unsubstituted alkylene groups having 1 to 5 carbon atoms and wherein n has a value of 5 to 15.

3. A composition accord to claims 1 or 2 wherein R to $R^3$ have the formula $-(CH_2)-_Y$ wherein Y is 3 and wherein n has a value of 3 to 10.

4. A composition according to either preceding claim characterised by an auxiliary complexing agent, selected from a transition metal ion which is capable of oxidation.

5. A composition according to claim 4 characterised by further comprising an organic acid having hydroxy or acid moieties attached to a carbon atom adjacent to a carboxylic acid group.

6. A composition according to claim 5 characterised in that the organic acid is selected from lactic, citric or ascorbic acids.

7. A composition according to any preceding claim characterised by the presence of a quaternary ammonium salt and/or an organic acid, and/or a betain compound.

8. A composition according to claim 7 characterised in that the quaternary ammonium salt is a substituted or unsubstituted aromatic quaternary ammonium salt, and/or the organic acid is selected from citric, ascorbic or lactic acids.

9. A composition according to claim 8 characterised in that the quaternary ammonium compound is dimethylbenzyl ammonium chloride, the complexing agent is the reaction product of citric ascorbic or lactic acid and further comprising a reactive salt of iron or copper and in that the carrier is a non-ionic or amphoteric surfactant.

10. A composition according to claim 1 comprising 20 to 80% by volume of a quaternary ammonium, 5 to 40% by volume of the polymer of formula 1 and up to 75% by volume of the carrier composition.

11. A composition according to any preceding claim characterised by a buffering agent to provide a pH of 5.5 to 10, and/or a cationic or non ionic wetting agent.

12. A composition according to any preceding claim characterised by containing an enzyme adapted to degrade odour emitting compounds.

13. A composition according to any preceding claim characterised in that at least one of the active components of the composition according to claim 1 is present as an acid or salt of low solubility in aqueous media.

14. An adsorbent article impregnated with a composition according to any preceding claim.

European Patent Office

# EUROPEAN SEARCH REPORT

Application Number

EP 88 30 0208

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-4 311 479 (D.J. FENN)<br>* Column 2, lines 56-58; claims 1,12,17 * | 1-14 | A 61 L 9/01 //<br>A 01 N 47/44<br>C 11 D 3/48 |
| Y | EP-A-0 041 448 (N. SALKIN)<br>* Page 3, lines 28-30; claims 1,3,6 * | 1-14 | |
| Y | EP-A-0 174 128 (I.C.I.)<br>* Page 2, lines 11-34; page 4, lines 15-17; claims 1,8,9 * | 1-14 | |
| A | EP-A-0 099 209 (SURGIKOS)<br>* Claims 1,7,8,10-15 * | 1-14 | |
| Y | EP-A-0 147 464 (MINATO SANGYO)<br>* Claims 1-4 * | 4-9 | |
| A | EP-A-0 180 309 (BAUSCH & LOMB) | | |

TECHNICAL FIELDS SEARCHED (Int. Cl.4)

A 61 L 9/01
A 01 N 47/44

The present search report has been drawn up for all claims

| Place of search | Date of completion of the search | Examiner |
|---|---|---|
| THE HAGUE | 12-09-1988 | PELTRE CHR. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document